(19) Europäisches Patentamt
European Patent Office
Office européen des brevets

(11) EP 1 886 713 A2

(12) **DEMANDE DE BREVET EUROPEEN**

(43) Date de publication:
**13.02.2008 Bulletin 2008/07**

(51) Int Cl.:
***A61Q 1/02*** *(2006.01)* ***A61K 8/97*** *(2006.01)*

(21) Numéro de dépôt: **07301067.0**

(22) Date de dépôt: **28.05.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **29.05.2006 FR 0651940**
**12.01.2007 FR 0752649**

(71) Demandeur: **L'ORÉAL**
**75008 Paris (FR)**

(72) Inventeurs:
- **Arnaud, Pascal**
**94240, L'HAY LES ROSES (FR)**
- **Feltin, Charlotte**
**75017, PARIS (FR)**
- **Bazelaire, Eve**
**94340, JOINVILLE-LE-PONT (FR)**

(74) Mandataire: **Le Coupanec, Pascale A.M.P.**
**Nony & Associés,**
**3 rue de Penthièvre**
**75008 Paris (FR)**

(54) **Procédé de coloration d'une peau foncée**

(57) La présente invention concerne un procédé de maquillage d'une peau foncée comprenant l'application sur ladite peau d'une composition comprenant dans un milieu physiologiquement acceptable au moins un système de coloration directe à l'état soluble, ladite composition possédant en outre une viscosité supérieure à 100 centipoises et un pouvoir couvrant, défini par le Contrast Ratio, inférieur à 80.

EP 1 886 713 A2

## Description

La présente invention vise à proposer des compositions cosmétiques à appliquer sur la peau et plus particulièrement des compositions destinées aux peaux foncées, y compris noires et métissées.

D'une manière générale, les produits cosmétiques et en particulier ceux destinés au maquillage visent à procurer un effet esthétique, fréquemment coloriel, au niveau du support du maquillage, voire un effet de camouflage à l'égard des défauts cutanés de la surface à maquiller.

Pour procurer de tels effets, les compositions cosmétiques associent généralement des charges minérales ou organiques et des agents de coloration.

Les charges sont classiquement utilisées pour modifier la rhéologie, ajuster la texture de la composition, en réduire la brillance et/ou à titre d'agent matifiant destiné notamment à procurer un effet de couvrance avantageux pour camoufler les imperfections cutanées. Ces charges sont donc classiquement choisies en fonction de leur propriété colorielle, leur couvrance ou encore leur géométrie qui permettent de diffuser la lumière de manière efficace. Elles se présentent généralement à l'état de particules de toutes formes, incolores ou blanches, minérales ou de synthèse et qui sont solides dans le milieu de composition quelque soit la température à laquelle la composition est fabriquée. Il s'agit plus particulièrement de silice, mica, talc et de kaolin. Ces charges sont classiquement utilisées à raison de 1 à 80 %, et notamment de 1 à 50 % en poids par rapport au poids total de la composition cosmétique.

En ce qui concerne les agents de coloration dont les pigments, ils sont plus particulièrement utilisés à titre d'opacifiant et/ou de colorant et sont généralement présents à une concentration suffisante pour procurer la couleur souhaitée.

Par pigments, il faut comprendre des particules blanches ou colorées, minérales ou organiques, insolubles dans la phase hydrophile ou lipophile liquide, destinées à colorer et/ou à opacifier la composition, la peau ou les muqueuses.

Les pigments d'oxydes de fer et les oxydes de titane sont connus depuis longtemps pour leurs propriétés couvrantes et colorantes superficielles et momentanées sur la peau. Ils sont généralement utilisés dans les produits de maquillage tels que les fonds de teint, les mascaras, les eye-liners et les rouges à lèvres. Leur taille moyenne peut varier en général entre 0,2 et 50 $\mu$m en fonction des effets de coloration recherchés et de l'application envisagée. On peut citer, par exemple, les compositions de maquillage les contenant telles que décrites dans les demandes de brevet WO 94/15580, WO 96/36309, WO 96/33690, WO 96/36323, FR-A-2754708.

Toutefois, ces compositions cosmétiques conventionnelles ne donnent pas totalement satisfaction lorsqu'elles sont appliquées sur une peau foncée. Ainsi, leur teneur importante en charge, à l'image du talc et du kaolin, et de pigments, tels que le $TiO_2$ par exemple, a pour effet de procurer sur ce type de support de maquillage, un effet grisâtre, voire cendreux, qui pour des raisons esthétiques évidentes est particulièrement indésirable.

Or, il est clair que la diminution de la quantité en charge et/ou en pigment qui pourrait être bénéfique en terme d'éclat de maquillage risque par ailleurs d'être préjudiciable à la couvrance parallèlement recherchée pour masquer les dyschromies par exemple.

De plus, l'effet coloriel procuré par ce type de composition conventionnelle est susceptible d'évoluer dans le temps et notamment de devenir inhomogène en réponse à une sécrétion de sébum et/ou de sueur.

En conséquence, il demeure à ce jour un besoin d'une composition de maquillage plus particulièrement destinée aux peaux foncées, qui permet de procurer à ce type de peaux une bonne homogénéité colorielle et une tenue satisfaisante dans le temps.

Le document EP 1 433 460 propose une composition de maquillage plus particulièrement destinée aux peaux foncées associant à des agents de coloration des particules réfléchissantes mais à des fins de procurer à ce type de peaux un effet éclaircissant et donc distinct de celui recherché selon l'invention.

Plus précisément, la présente invention concerne selon un de ses aspects, un procédé de maquillage d'une peau foncée comprenant l'application sur ladite peau d'une composition comprenant dans un milieu physiologiquement acceptable au moins un système de coloration directe à l'état soluble, ladite composition possédant une viscosité supérieure à 100 centipoises, et un pouvoir couvrant, défini par le Contrast Ratio, inférieur à 80.

La présente invention vise également selon un autre de ses aspects, un procédé de maquillage d'une peau foncée comprenant l'application sur ladite peau d'une composition comprenant dans un milieu physiologiquement acceptable au moins agent de coloration directe à l'état soluble, ladite composition possédant un pouvoir couvrant défini par le Contrast Ratio, inférieur à 80, et un pH supérieur à 5.

Au sens de la présente invention, on entend par "peaux foncées", des peaux dont la clarté moyenne L* mesurée sur le front, les pommettes et le menton dans l'espace colorimétrique CIE 1976, est inférieure à 55. La saturation C* peut être comprise par exemple entre 10 et 30, notamment entre 12 et 28. Les valeurs d'angle de teinte h en ° peuvent être comprises par exemple entre 38° environ et 54° environ. Les valeurs de clarté L* peuvent être inférieures ou égales à 50, voire 45 ou 40 pour les peaux les plus sombres, tout en pouvant rester, pour la plupart des peaux, supérieures à 30.

Des peaux foncées sont rencontrées par exemple parmi les populations africaine, afro-américaine, hispano-américaine, indienne et maghrébine.

Ces peaux peuvent encore être classées sur la base de leur réactivité aux effets du rayonnement solaire selon l'échelle proposée par FITZPATRICK.

Selon cette échelle, les différentes peaux ethniques existantes peuvent être distinguées selon les types suivants :

| Type | Réactivité de la peau | Origine |
|---|---|---|
| I | Brûle toujours, ne bronze jamais | Celte |
| II | Brûle toujours, bronze peu | Germanique |
| III | Brûle modérément, bronze progressivement | Européen |
| IV | Brûle faiblement, bronze très facilement | Méditerranéen |
| V | Brûle rarement, bronze intensément | Moyen Orient - Sud Américain |
| VI | Ne brûle jamais, fortement pigmentée | Africain |

Les peaux foncées qui font plus particulièrement l'objet de la présente invention appartiennent aux types IV à VI.

Selon un mode de réalisation avantageux, les compositions considérées selon l'invention procurent une variation de clarté $\Delta L$ négative variant de -20 à -1 et une variation de saturation $\Delta C^*$ variant de 0,5 à 20 lorsqu'elles sont appliquées sur la zone B11+ d'une carte de contraste telle que définie dans le document EP 1 433 462.

Cette carte présente 3 fois cinq zones ayant respectivement pour coordonnées colorimétriques, à 15 % près, voire à 12,5 %, mieux à 10 % près, voire à 7,5 % près, voire à 5 % près, pour L* et h, et à 25 % près, mieux à 20 %, encore mieux 15 %, voire à 10 %, ou voire 5 % près, pour C*,

- première zone (B11+) : L* = 36,7 C* = 19,81 h = 47,34°,
- deuxième zone (B11): L* = 38,43 C* = 21,76 h = 46,51°,
- troisième zone (B12): L* = 35,66 C* = 19,78 h = 46,32°,
- quatrième zone (B12+) : L* = 32,98 C* = 17,29 h = 44,64°,
- cinquième zone (XXX) : L* = 29,63 C* = 15,06 h = 40,34°,
- première zone (C9) : L* = 45,04 C* = 25,18 h = 53,27°,
- deuxième zone (B12): L* = 35,66 C* = 19,78 h = 46,32°,
- troisième zone (C11): L* = 38,73 C* = 21,94 h = 50,18°,
- quatrième zone (C10): L* = 42,19 C* = 24,18 h = 51,94°,
- cinquième zone (C8): L* = 48,06 C* = 25,97 h = 53,09°,
- première zone (D6) : L* = 54,08 C* = 26,70 h = 57,35°,
- deuxième zone (C11): L* 5 = 38,73 C* = 21,94 h = 50,18°,
- troisième zone (D8) : L* = 47,94 C* = 26,18 h = 56,82°,
- quatrième zone (D7) : L* = 51,79 C* = 27,21 h = 57,09°,
- cinquième zone (D5): L* = 57,61 C* = 26,22 h = 55,09°,

Les mesures des coordonnées trichromatiques de la composition, L*, C* et h sont effectuées avec un spectrocolorimètre de marque MINOLTA®, de référence CM3700d, en mode réflexion, spéculaire inclus, petite ouverture SAV 5 mm.

On dépose un film de composition à l'aide de l'applicateur automatique de film thermostaté BRAVE INSTRUMENTS et d'un étaleur calibré BRAVE INSTRUMENTS, épaisseur du film : 20 µm, sur un film transparent IMPEGA transparent pour photocopieur. On laisse sécher 10 minutes à 37 °C dans une étuve, et l'on superpose ce film à la carte de contraste. On mesure l'écart colorimétrique entre la carte de contraste à travers le film transparent et la carte de contraste à travers le film ainsi recouvert de composition.

$\Delta$ = (carte de contraste + film de crème sur le film transparent) – (carte de contraste avec le film transparent).

## POUVOIR COUVRANT

Avantageusement, les compositions cosmétiques selon l'invention se caractérisent par un pouvoir couvrant, défini par le Contrast Ratio, inférieur à 80, en particulier inférieur ou égal à 50.

Ce pouvoir couvrant peut être mesuré par la méthode suivante.

La mesure est réalisée sur un film de composition étalé sur une carte de contraste Erichsen, type 24/5, présentant un fond noir et un fond blanc.

Dans le cas d'un stick, la composition est préalablement malaxée de façon à obtenir une pâte visqueuse.

Dans le cas d'une poudre, 50 parties en poids de la poudre sont malaxées avec 50 parties en poids de diméthicone (DC 200 Fluid 5CST de DOX CORNING) de façon à obtenir une pâte visqueuse.

Le film est étalé avec une épaisseur de 50 μm et les coordonnées trichromatiques (X, Y, Z) sont mesurées à l'aide d'un colorimètre CM-2002 ou CR-3700.

Des étalements similaires sont réalisés sur deux autres cartes de contraste et trois mesures sont effectuées sur chaque carte. La moyenne correspondant à ces neuf mesures est ensuite calculée.

Le pouvoir couvrant est égal à 100 x Yn/Yb où Yn est la valeur moyenne de Y sur fond noir et Yb est la valeur moyenne de Y sur fond blanc.

Plus la valeur du rapport est élevée, plus le pouvoir couvrant est important.

## SYSTEME DE COLORATION DIRECTE

Par "système de coloration directe" on désigne un agent de coloration ou mélange d'agents de coloration qui dès leur mise en contact avec le support de maquillage, généralement la peau, procure instantanément un effet de coloration associé.

Sont ainsi exclus de cette définition, les composés qui exercent une action autobronzante sur la peau humaine à l'image des dérivés présentant un groupement cétone comme des hydroxyméthylcetones et en particulier la dihydroxyacétone (DHA) et également le méthylglyoxane. En effet, par opposition au système de coloration directe selon l'invention, ces composés provoquent un effet coloriel non immédiat.

Les inventeurs ont ainsi constaté de manière inattendue, que l'utilisation d'un système de coloration directe tel que défini ci-dessus sous une forme soluble, permet d'obtenir un résultat homogénéisant et éclatant en terme de maquillage sans caractère terne ni cendreux.

Par ailleurs, la mise en oeuvre d'un tel système permet d'augmenter sensiblement la tenue du maquillage dans le temps, notamment en terme de résistance à la sueur et/ou au sébum.

Ainsi, les compositions cosmétiques considérées selon l'invention permettent de conférer aux peaux foncées une coloration ayant une très bonne tenue et qui se distingue avantageusement des colorations plus superficielles et susceptibles d'évoluer, procurées par des compositions de maquillage conventionnelles et qui sont généralement formulées pour l'essentiel à partir de pigments en dispersion.

Le ou les agents de coloration formant le système de coloration directe considéré dans le cadre de la présente invention peuvent être présents à une teneur allant de 0,001 à 5 %, notamment de 0,01 à 3 %, et plus particulièrement de 0,025 à 1 % en poids par rapport au poids total de la composition.

Le système de coloration directe peut représenter au moins 30 % en poids, en particulier au moins 50 % en poids, notamment au moins 65 % en poids, plus particulièrement au moins 70 % en poids, voire au moins 75 % en poids et plus particulièrement au moins 90 % en poids de l'ensemble des agents de coloration présents dans la composition.

Selon un mode de réalisation particulier, ce système de coloration directe figure l'intégralité des agents de coloration de ladite composition.

Comme précisé précédemment, le système de coloration directe se trouve à un état solubilisé au sein de la composition par opposition aux formulations conventionnelles dans lesquelles les agents de coloration sont pour l'essentiel formulés à un état dispersé.

Les agents de coloration formant le système de coloration directe peuvent être donc liposolubles ou hydrosolubles. Avantageusement ils sont hydrosolubles.

Les agents de coloration du système de coloration directe peuvent être synthétiques ou naturels.

Il peut s'agir de colorants organiques ou minéraux.

Les colorants liposolubles, synthétiques ou naturels sont, par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les colorants hydrosolubles synthétiques ou naturels sont par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétanine (betterave), le carmin, la chlorophylline cuivrée, le bleu de

méthylène, les anthocyanines (enocianine, carotte noire, hibiscus, sureau), le caramel, la riboflavine.

Les colorants peuvent encore être choisis parmi la juglone, la lawsone, les extraits de soja fermenté, d'algues, de champignons, de micro-organismes. Les sels de Flavylium non substitués en position 3 tels que par exemple ceux décrits dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Dans un mode de réalisation particulier, le système de coloration directe comprend au moins un agent de coloration naturel, notamment tel que défini ci-dessus, en particulier hydrosoluble.

La composition peut comprendre en outre des pigments et/ou nacres à l'état dispersés sous réserve que ceux-ci n'affectent pas les propriétés attendues de la composition.

Avantageusement, ces agents de coloration insolubles peuvent être présents à raison de moins de 8 % en poids, plus particulièrement de moins de 5 % et notamment de moins de 3 % en poids par rapport au poids total de la composition.

A titre illustratif et non limitatif des pigments minéraux, on peut plus particulièrement citer les oxydes métalliques jaunes, rouges, bruns comme par exemple les oxydes de fer.

Comme poudres métalliques, on peut citer la poudre de cuivre.

Les pigments mis en oeuvre dans le cadre de la présente invention, peuvent être utilisés soit sous leur forme brute ou sous une forme prétraitée, notamment en leur surface.

A titre représentatif de ces traitements de surface, on peut notamment citer celui consistant à traiter le pigment avec un agent hydrophobe et oléofuge de type dérivé phosphate perfluoroalkyle comme décrit dans le brevet EP 1 086 683.

A titre illustratif des pigments convenant plus particulièrement à l'invention, on peut notamment citer les oxydes de fer brun et de fer jaune, enrobés de phosphate de perfluoroalkyle et l'oxyde de titane traité alumine, enrobé de phosphate de perfluoroalkyle, comme en particulier les pâtes pigmentaires commercialisées, sous les dénominations commerciales YELLOW IRON OXYDE COVAFLUOR, PF5 YELLOW 601 (jaune) et PF5 R516L (rouge) par la société DAITO, sous les dénominations commerciales FA50DRF, FA50DYF, FA65DF et FA65DBF par la société KOBO.

Par "nacres", il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Les compositions selon l'invention peuvent également comprendre à titre d'agent de coloration supplémentaire, un ou plusieurs composés qui exercent une action autobronzante sur la peau humaine à l'image des dérivés présentant

un groupement cétone comme des hydroxyméthylcetones et en particulier la dihydroxyacétone (DHA) et également le méthylglyoxane.

**MILIEU PHYSIOLOGIQUEMENT ACCEPTABLE**

Par "milieu physiologiquement acceptable", on désigne un milieu non toxique et susceptible d'être appliqué sur la peau d'êtres humains. Le milieu physiologiquement acceptable est généralement adapté à la nature de la peau sur laquelle doit être appliquée la composition ainsi qu'à la forme sous laquelle la composition est destinée à être conditionnée, notamment fluide à la température ambiante et sous pression atmosphérique.
Comme précisé précédemment, les compositions de l'invention sont plus particulièrement des compositions destinées au maquillage de la peau et notamment de la peau du visage. Il peut ainsi s'agir de compositions de base et/ou de type fond de teint.
Elles peuvent être solides ou liquides et sont avantageusement formulées sous une forme fluide. Ainsi elles peuvent se présenter sous une forme anhydre ou sous la forme d'un gel, d'émulsion directe, inverse ou multiple associant au moins une phase aqueuse et au moins une phase grasse.
Comme précisé précédemment, elles possèdent avantageusement une viscosité supérieure à 100 centipoises, en particulier supérieure ou égale à 300 centipoises.
Cette viscosité peut être mesurée par la méthode suivante.
La mesure est réalisée à l'aide d'un viscosimètre de type Rheomat RM 180. Le produit est versé dans le godet adapté au mobile utilisé, selon la fluidité du produit. La viscosité est mesurée après 10 minutes de rotation, à une vitesse de 75 $s^{-1}$, et à température ambiante.

**Phase aqueuse**

Les compositions selon l'invention peuvent comprendre au moins un milieu aqueux, constituant une phase aqueuse, qui peut former la phase continue de la composition considérée.
La phase aqueuse peut être constituée essentiellement d'eau.
Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.
Selon un mode de réalisation particulier, le solvant type monoalcool inférieur dans le cadre de la présente invention peut représenter de 1 à 70 %, et préférentiellement de 2 à 50 % en poids de la composition mise en oeuvre dans le procédé revendiqué.
A titre d'exemple de monoalcool convenant à la mise en oeuvre de l'invention, on peut mentionner les composés hydrocarbonés saturés comprenant une unique fonction hydroxyle et comprenant de 2 à 15 atomes de carbone, en particulier de 4 à 12 atomes de carbone.
A titre d'exemple de monoalcool convenant à l'invention, on peut citer, de manière non limitative l'éthanol, le propanol, l'isopropanol, le butanol, le pentanol, le propanol, l'hexanol, l'heptanol, l'octanol, le décanol et le dodécanol.
Par le terme "solvant polyhydroxylé", on entend couvrir tout composé hydrocarboné comprenant au moins deux fonctions hydroxyle et comprenant de 4 à 40 atomes de carbone, en particulier de 6 à 36 atomes de carbone, en particulier de 8 à 32 atomes de carbone, en particulier de 16 à 28 atomes de carbone, et plus particulièrement de 18 à 24 atomes de carbone.
Les chaînes hydrocarbonées peuvent, le cas échéant, être interrompues par la présence d'au moins un hétéroatome, et notamment un atome d'oxygène.
Le polyol convenant à la mise en oeuvre de la présente invention peut être, notamment, choisi parmi les alcools linéaires, ramifiés, cycliques ou polycycliques, saturés ou insaturés.
Ainsi, le polyol peut être choisi par exemple parmi un diol, un triol, un tétraol, ou un pentaol, ou un de leurs esters.
Le polyol peut être un diol, ou un de ses esters, par exemple choisi parmi un dimère d'alcool gras, un mono- ou poly-glycérol, un mono- ou poly-alkylène en $C_{2-4}$ glycol, le 1,4 butanediol, et le pentaérythritol.
A titre d'exemple de diol pouvant également convenir à la mise en oeuvre de l'invention, on peut citer, de manière non exhaustive, le butanediol, le pentanediol, le propanediol, l'hexanediol, l'hexylèneglycol, l'heptanediol, l'octanediol, le nonanediol, le décanediol, l'un-décanediol, le dodécanediol, le tridécanediol, le tétradécanediol, le pentadécanediol, l'hexadécanediol, le nonadécanediol, l'octadecènediol, le cyclohexanediol, le 1,1'-oxydipropanediol, le diglycérol, l'érythritol, le pentaérythritol, le xylitol, le sorbitol, le phytantriol (3,7,11,15-tetraméthyl-1,2,3-trihydroxy-hexadecane) l'éthylèneglycol, le xylèneglycol et leurs isomères.
Convient tout particulièrement comme mélange, un mélange à base au moins d'éthanol et de propylène glycol ou de dipropylène glycol ou de butylène glycol.

La phase aqueuse (eau et éventuellement le(s) solvant(s) organique(s) miscible(s) à l'eau) peut être présente, à une teneur allant de 1 % à 99 % en poids, notamment allant de 3 % à 80 % en poids, et en particulier allant de 5 % à 60 %, en poids par rapport au poids total de la composition considérée.

## Phase grasse

La composition selon l'invention peut comporter une phase grasse et notamment au moins un corps gras liquide à température ambiante (25 °C) et/ou un corps gras solide à température ambiante tel que les cires, les corps gras pâteux, les gommes et leurs mélanges. La phase grasse peut en outre contenir des solvants organiques lipophiles.
La composition peut posséder par exemple une phase grasse continue, pouvant contenir moins de 5 % d'eau, notamment moins de 1 % d'eau par rapport à son poids total et en particulier être sous forme anhydre.
La phase grasse de la composition selon l'invention peut notamment comprendre, à titre de corps gras liquide, au moins une huile volatile ou non volatile ou un de leurs mélanges.
Par "huile volatile", on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).
Par "huile non volatile", on entend une huile restant sur la peau à température ambiante et pression atmosphérique au moins plusieurs heures et ayant notamment une pression de vapeur inférieure à 0,01 mm de Hg (1,33 Pa).
Ces huiles volatiles ou non volatiles peuvent être des huiles hydrocarbonées, des huiles siliconées, ou leurs mélanges. On entend par "huile hydrocarbonée", une huile contenant principalement des atomes d'hydrogène et de carbone et éventuellement des atomes d'oxygène, d'azote, de soufre, de phosphore.
Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en$C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en $C_8$-$C_{16}$ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.
Comme huiles volatiles, on peut aussi utiliser les silicones volatiles, comme par exemple les huiles de silicones linéaires ou cycliques volatiles, notamment celles ayant une viscosité $\leq$ 8 centistokes (8 x $10^{-6}$ $m^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
L'huile volatile peut être présente dans une composition selon l'invention à une teneur allant de 0,1 à 98 % en poids, notamment de 1 % à 65 % en poids, et en particulier de 2 % à 50 % en poids, par rapport au poids total de la composition.
Les huiles non volatiles peuvent notamment être choisies parmi les huiles hydrocarbonées fluorées et/ou siliconées non volatiles.
Comme huile hydrocarbonée non volatile, on peut notamment citer :

- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule $R_1COOR_2$ dans laquelle $R_1$ représente le reste d'un acide gras

linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles non volatiles peuvent être présentes dans une composition selon l'invention en une teneur allant de 0,01 à 90 % en poids, notamment de 0,1 à 85 % en poids, et en particulier de 1 % à 70 % en poids, par rapport au poids total de la composition.

Plus généralement, le corps gras liquide peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Ainsi, une composition selon l'invention peut comprendre au moins un composé gras pâteux à température ambiante.

Par "corps gras pâteux" au sens de l'invention, on entend des corps gras ayant un point de fusion allant de 20 à 55 °C, de préférence 25 à 45 °C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans une composition selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55 °C et leurs mélanges. On peut également utiliser des esters d'acides ou d'alcools gras, notamment ceux ayant 20 à 65 atomes de carbone (point de fusion de l'ordre de 20 à 35 °C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycéride d'origine végétale, on peut utiliser les dérivés d'huile de ricin hydrogénée, tels que le "THIXINR" de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société Dow Corning sous les noms commerciaux de DC2503® et DC25514®, et leurs mélanges.

Le corps gras pâteux peut être présent dans une composition selon l'invention en une teneur allant de 0,01 à 50 % en poids, de préférence allant de 0,1 à 45 % en poids, et mieux allant de 0,2 % à 30 % en poids, par rapport au poids total de ladite composition.

La composition selon l'invention peut comprendre en outre une cire. La cire peut être solide à température ambiante (25 °C), à changement d'état solide/liquide réversible, ayant une température de fusion supérieure à 30 °C pouvant aller jusqu'à 200 °C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, les cires microcristallines, et leurs mélanges.

En particulier, la cire peut être présente sous forme d'émulsion cire-dans-eau.

La cire peut être présente dans une composition selon l'invention en une teneur allant de 0,01 % à 50 % en poids, en particulier de 0,1 % à 30 % en poids, et notamment de 0,2 % à 20 % en poids, par rapport au poids total de la composition.

**Agents tensioactifs**

La composition peut en outre contenir des agents tensioactifs émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document "Encyclopedia of Chemical Technology, KIRK-OTHMER", volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition de type fond de teint selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges,
- parmi les tensioactifs anioniques : les acides gras en $C_{16}$-$C_{30}$ neutralisés par les amines, l'ammoniaque ou les sels alcalins, et leurs mélanges.
- Parmi les tensioactifs siliconés choisis parmi les composés de formule générale (I) suivante :

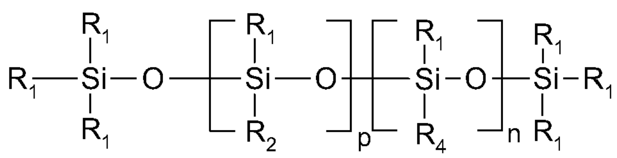

(I)

formule dans laquelle :

- $R_1$ et $R_4$, identiques ou différents, représentent un atome d'hydrogène, un radical alkyle linéaire ou ramifié en $C_1$-$C_{30}$ ou un radical phényle,
- $R_2$, identiques ou différents, représentent $-(C_xH_{2x})-(OC_2H_4)_a-(OC_3H_6)_b-OR_3$ ,
- $R_3$, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 1 à 1000,
- p varie de 1 à 30,
- a varie de 1 à 50,
- b varie de 1 à 50,
- x varie de 1 à 5.

Le poids moléculaire moyen en nombre de cet émulsionnant siliconé est en général supérieur ou égal à 15000 et de préférence compris entre 20 000 et 40 000.

Une première famille de polyalkyl polyéther siloxanes convenant particulièrement bien aux compositions selon l'invention est celle des composés répondant à la formule (I) ci-dessus pour lesquels les radicaux $R_1$ et $R_4$ sont identiques et représentent tous des radicaux méthyle et le radical $R_3$ représente l'hydrogène.

A titre d'exemple d'émulsionnant siliconé appartenant à cette famille, on peut citer le poly diméthyl/méthyl siloxane oxyéthyléné oxypropyléné (OE/OP 18/18) pour lequel n est 396 et p est 4, de poids moléculaire en nombre supérieur à 30 000 (nom CTFA : Cyclométhicone 90 % Dimethicone copolyol 10 %) vendu sous la dénomination commerciale de "Silicone Q2-3225C" par la société Dow Corning.

Dans la définition ci-dessus et dans la suite du texte, OE représente une mole d'oxyde d'éthylène et OP représente une mole d'oxyde de propylène.

Une deuxième famille de polyalkyl polyéther siloxanes convenant particulièrement bien aux compositions selon l'invention est celle des composés répondant à la formule (I) ci-dessus pour lesquels les radicaux $R_1$ représentent tous

des radicaux méthyle et les radicaux R4 représentent tous des radicaux lauryle.

Un émulsionnant siliconé particulièrement préféré de cette deuxième famille est le poly méthyllauryl/méthyl siloxane oxyéthyléné oxypropyléné (OE/OP 18/18) pour lequel n est 35 et p est 3, de poids moléculaire en nombre supérieur à 25 000 (nom CTFA : Lauryl méthicone copolyol 91 %, Isostéaryl alcohol 9 %) vendu sous la dénomination commerciale "DC Q2-5200" par la société Dow Corning.

Un émulsionnant siliconé tout particulièrement préféré pour les compositions selon l'invention est une silicone oxyalkyléne substituée en α-ω à structure linéaire, substitué aux deux extrémités de la chaîne principale par des groupements oxyalkylènes reliés aux atomes de Si par l'intermédiaire d'un groupement hydrocarboné. On choisira plus particulièrement une silicone répondant à la formule générale (II) suivante :

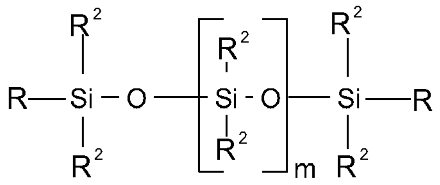

(II)

dans laquelle : R = $-(CH_2)s\ O-(C_2H_4O)t\ (C_3H_6O)uR_1$
où :

- $R_1$ représente H, $CH_3$ ou $CH_2CH_3$,
- s est un entier allant de 1 à 5, t varie de 1 à 100, u varie de 0 à 50,
- les unités ($C_2H_4O$) et ($C_3H_6O$) pouvant être réparties de façon aléatoire ou par blocs,

ou

- les radicaux $R^2$ représentent un radical alkyle en $C_1$-$C_3$ ou un radical phényle,
- $5 \leq m \leq 300$,

De préférence, la silicone oxyalkylénée substituée en α-ω utilisée selon la présente invention répond à la formule générale (II) pour laquelle tous les radicaux $R^2$ sont des radicaux méthyles et :

- s va de 2 à 4,
- t va de 3 à 100,
- m va de 50 à 200.

De préférence encore, le poids moléculaire moyen de R va de 800 à 2600.

De préférence, le rapport en poids des unités $C_2H_4O$ par rapport aux unités $C_3H_6O$ va de 100:10 à 20:80. De façon avantageuse, ce rapport est d'environ 42/58.

De préférence encore, $R_1$ est le groupe méthyle.

De façon plus préférentielle encore, l'émulsion selon l'invention comprend la silicone oxyalkylénée en α-ω de formule suivante :

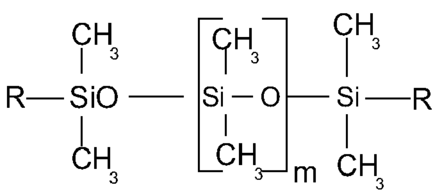

dans laquelle:

- m = 100,
- R = $(CH_2)_3$-O-$(C_2H_4O)$x-$(C_3H_6O)$y-$CH_3$, où t va de 3 à 100, u va de 1 à 50, le rapport en poids du nombre de $C_2H_4O$ sur le nombre de $C_3H_6O$ étant d'environ 42/58, le poids moléculaire moyen de R allant de 800 à 1000.

Parmi les produits du commerce pouvant contenir tout ou partie des silicones oxyalkylénées substituées en $\alpha$-$\omega$ utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.
L'émulsionnant polyalkyl polyéther siloxane tel que défini ci-dessus est utilisé selon l'invention en une proportion préférentielle allant de 0,1 à 30 % et plus particulièrement de 0,5 à 10 % en poids par rapport au poids total de l'émulsion.

**<u>Polymère filmogène</u>**

La composition selon l'invention peut comprendre, en outre, au moins un polymère filmogène.
Selon la présente invention, on entend par "polymère filmogène", un polymère apte à former à lui seul ou en présence d'un agent auxiliaire de filmification, un film continu et adhérent sur la peau.
On utilise de préférence un polymère filmogène apte à former un film hydrophobe, c'est-à-dire un polymère dont le film a une solubilité dans l'eau à 25 °C inférieure à 1 % en poids.
Le polymère filmogène peut notamment être au moins un polymère choisi parmi le groupe comprenant :

- les polymères filmogènes hydrosolubles,
- des dispersions aqueuses de particules de polymères filmogènes hydrodispersibles, encore appelées "latex" ; dans ce cas, la composition doit comprendre une phase aqueuse,
- des polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées "NAD".

Le polymère filmogène peut être présent dans une composition selon l'invention en une teneur en matières sèches allant de 0,01 % à 20 % en poids par rapport au poids total de la composition et notamment de 0,5 % à 10 % en poids.
Parmi les polymères filmogènes utilisables selon l'invention, on peut citer les polymères synthétiques, de type radicalaire ou de type polycondensat, les polymères d'origine naturelle, et leurs mélanges.
Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaire peuvent être notamment des polymères ou des copolymères, vinyliques, notamment des polymères acryliques.
Comme dispersion aqueuse de polymère filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou bien encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450® , SANCURE 875®, SANCURE 861®, SANCURE 878®, SANCURE 2060® par la société GOODRICH, IMPRANIL 85® par la société BAYER, AQUAMERE H-1511® par la société HYDROMER.
Comme exemples de polymères filmogènes hydrosolubles, on peut citer les protéines, les polymères de chitine ou de chitosane anioniques, cationiques, amphotères ou non-ioniques, les polymères de cellulose, les polymères ou copolymères acryliques, les polymères vinyliques, les polymères d'origine naturelle, éventuellement modifiés, et leurs mélanges.
Le polymère filmogène peut être également présent sous forme de particules, stabilisées en surface, dispersées dans la phase grasse liquide. Des dispersions de polymère filmogène dans la phase grasse liquide, en présence d'agents stabilisants, sont notamment décrites dans les documents EP-A-749746, EP-A-923928, EP-A-930060.
Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.
La composition selon l'invention peut en outre comprendre un agent auxiliaire de filmification favorisant la formation d'un film avec le polymère filmogène. Un tel agent de filmification peut être choisi parmi tous les composés

connus de l'homme du métier comme étant susceptibles de remplir la fonction recherchée, et notamment être choisi parmi les agents plastifiants et les agents de coalescence.

## CHARGE

Bien entendu, il demeure possible introduire dans la composition selon l'invention une ou plusieurs charges conventionnelles sous réserve que celles-ci soient utilisées en une quantité telle qu'elles ne viennent pas affecter l'effet esthétique recherché par les compositions considérées selon l'invention, c'est-à-dire ne confèrent pas outre mesure un aspect grisâtre à la peau maquillée lorsque celle-ci est revêtue d'une composition conforme à l'invention. L'homme de l'art est à même, de par ses connaissances, de procéder à cet ajustement.

Ces charges peuvent être minérales ou organiques de toute forme, plaquettaires, sphériques ou oblongues, quelque soit la forme cristallographique (par exemple feuillet, cubique, hexagonale, orthorhombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) ou de polyméthacrylate de méthyle (Covabead de Wackherr), les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

En l'occurrence, les compositions conformes à l'invention peuvent contenir moins de 15 % voire moins de 5 % en poids en charge, voire peuvent être exemptes en charge.

Il peut être avantageux de privilégier le choix de charges, transparentes, comme par exemple la silice pyrogénée, le polyméthacrylate de méthyle (PMMA).

La composition considérée selon l'invention se présente généralement sous la forme d'une composition de base de maquillage, d'un fond de teint notamment à appliquer sur le visage ou le cou, d'un produit anti-cernes, d'un correcteur de teint, d'une crème teintée ou base de maquillage pour le visage ou d'une composition de maquillage pour le corps.

La composition selon l'invention peut se présenter sous la forme d'un fluide par exemple pâteux ou liquide. Elle peut se présenter sous forme de pâte souple, d'un onguent, d'une pommade solide ou fluide de type crème. Par exemple, elle peut être une émulsion huile-dans-eau, eau-dans-huile ou multiple, une émulsion solide notamment de type eau dans huile, un gel notamment anhydre, solide ou souple et même sous forme biphasique.

Elle peut aussi se présenter sous une forme solide, par exemple pulvérulente, compactée ou coulée ou sous forme stick.

L'invention pourra être mieux comprise à la lecture de la description détaillée qui va suivre, d'exemples de mise en oeuvre non limitatifs de celle-ci.

## EXEMPLES

Exemple 1 : Composition cosmétique de type émulsion huile-dans-eau colorée

| | | | |
|---|---|---|---|
| A | - | Mélange tartrate de dialkyle (C14-15 linéaire), alcool cétylstéarylique, alcool laurylique oxyéthylène (25 OE) oxypropylène (2) (COSMACOL PSE de SASOL) | 1,500 |
| | - | Mélange mono/distéarate de glycéryle/stéarate de polyéthylène glycol (100 OE) (ARLACEL 165 FL d'UNIQUEMA) | 2,000 |
| | - | Alcool stéarylique (C18 95 %) (LANETTE 18 de COGNIS) | 1,000 |
| | - | Cyclohexa diméthylsiloxane (DOW CORNING 246 FLUID de DOW CORNING) | 7,500 |
| | - | Isoparaffine (6-8 moles d'isobuthylène) hydrogénée (PARLEAM de NOF CORPORATION) | 2,500 |
| | - | P-hydroxybenzoate de butyle (distribué par UENO FINE CHEMICALS) | 0,150 |
| | - | 4-méthoxycinnamate de 2-éthyl hexyle protégé (PARSOL MCX de DSM NUTRITIONAL PRODUCTS) | 1,000 |
| B1 | - | Eau | 52,500 |
| | - | P-hydroxybenzoate de méthyle (distribué par NIPA) | 0,250 |

(suite)

| | | | |
|---|---|---|---|
| | - | Chlorphénésine (de DENK (MERCK)) | 0,250 |
| B2 | - | Eau | 16,350 |
| | - | Sel di-sodique de tartrazine (CI : 19140) (08005 FD et C YELLOW 5 de LCW (SENSIENT) | 0,255 |
| | - | Sel di-sodique du jaune brillant FCF (CI : 15985) (37001 FD & C YELLOW N°6 d'UNIVAR) | 0,302 |
| | - | Sel di-sodique de vert brillant FCF (CI : 42053) (06503 FD & C GREEN 3 de LCW (SENSIENT) | 0,035 |
| | - | Sel di-sodique de fuchsine acide D (CI :17200) (K7057 D et C RED 33 de LCW SENSIENT) | 0,158 |
| C | - | Eau | 1,000 |
| | - | Digluconate de chlorhexidine en solution (chlorhexidine digluconate 20% solution BP/PH. EUR. de SCHUTZ DISHMAN BIOTECH) | 0,250 |
| D | - | Eau | 9,750 |
| | - | Hyaluronate de sodium (PM : 1.100.000) en poudre (CRISTALHYAL de SOLIANCE) | 0,250 |
| E | - | Amidon de maïs estérifié par anhydride octénylsuccinique, sel d'aluminium (DRY FLO PLUS (28-1160) (EUROPE) de NATIONAL STARCH) | 3,000 |

Mode opératoire :

On pèse A dans un bécher de 400 mL qui est mis au bain marie à 75 °C jusqu'à dissolution complète.

On pèse les différents constituants de la phase B1 dans le bécher principal (800 ml) et de la phase B2 dans un bécher de 250 ml puis on les met au bain marie à 75 °C jusqu'à dissolution complète. Quand les 2 phases sont bien dissoutes B2 est ajouté dans B1 sous agitation magnétique.

La phase D (gel) se prépare en parallèle au Raynerie.

L'émulsion se fait à 75 °C. Pour cela, on verse la phase grasse A dans la phase aqueuse B, en augmentant au fur et à mesure la vitesse d'agitation jusqu'à 1500 tr/mn. Il doit toujours y avoir un petit vortex. L'agitation mécanique est maintenue pendant 10 minutes.

La température est ensuite descendue à 45 °C, et la phase C est ajoutée (agitation pendant 5 minutes), puis la phase D (agitation pendant 5 min), puis la phase E (agitation pendant 10 minutes).

Cette base de coloration permet d'obtenir sur la peau foncée une teinte marron neutre. Le pouvoir couvrant mesuré (Contrast Ratio) avec cette base de coloration est de 24,23 et sa viscosité est de 346 centipoises.

Exemple 2 : Composition cosmétique de type émulsion huile-dans-eau colorée

| | | | |
|---|---|---|---|
| A | - | Mélange tartrate de dialkyle (C14-15 linéaire), alcool cétylstéarylique, alcool laurylique oxyéthylène (25 OE) oxypropylène (2) (COSMACOL PSE de SASOL) | 1,500 |
| | - | Mélange mono/distéarate de glycéryle/stéarate de polyéthylène glycol (100 OE) (ARLACEL 165 FL d'UNIQUEMA) | 2,000 |
| | - | Alcool stéarylique (C18 95 %) (LANETTE 18 de COGNIS) | 1,000 |
| | - | Cyclohexa diméthylsiloxane (DOW CORNING 246 FLUID de DOW CORNING) | 7,500 |
| | - | Isoparaffine (6-8 moles d'isobuthylène) hydrogénée (PARLEAM de NOF CORPORATION) | 2,500 |
| | - | P-hydroxybenzoate de butyle (distribué par UENO FINE | 0,150 |
| | - | CHEMICALS) 4-méthoxycinnamate de 2-éthyl hexyle protégé (PARSOL MCX de DSM NUTRITIONAL PRODUCTS) | 1,000 |
| B1 | - | Eau | 52,500 |
| | - | P-hydroxybenzoate de méthyle (distribué par NIPA) | 0,250 |
| | - | Chlorphénésine (de DENK (MERCK)) | 0,250 |
| B2 | - | Eau | 16,129 |
| | - | Sel di-sodique de ponceau SX (CI : 14700) (07004 FD et C RED 4 de LCW (SENSIENT)) | 0,385 |
| | - | Sel di-sodique du jaune brillant FCF (CI: 15985) (37001 FD & C YELLOW N° 6 d'UNIVAR) | 0,500 |
| | - | Sel di-sodique de vert brillant FCF (CI : 42053) (06503 FD & C GREEN 3 de LCW (SENSIENT) | 0,048 |

(suite)

| | | | |
|---|---|---|---|
| | - | Sel di-sodique de fuchsine acide D (CI :17200) (K7057 D et C RED 33 de LCW SENSIENT) | 0,038 |
| C | - | Eau | 1,000 |
| | - | Digluconate de chlorhexidine en solution (chlorhexidine digluconate 20 % solution BP/PH. EUR. de SCHUTZ DISHMAN BIOTECH) | 0,250 |
| D | - | Eau | 9,750 |
| | - | Hyaluronate de sodium (PM : 1.100.000) en poudre (CRISTALHYAL de SOLIANCE) | 0,250 |
| E | - | Amidon de maïs estérifié par anhydride octénylsuccinique, sel d'aluminium (DRY FLO PLUS (28-1160) (EUROPE) de NATIONAL STARCH) | 3,000 |

Le mode opératoire est le même que celui de l'exemple 1.

Cette base de coloration permet d'obtenir sur une peau foncée une teinte marron rouge. Le pouvoir couvrant mesuré (Contrast Ratio) avec cette base de coloration est de 25,5 et sa viscosité est de 490 centipoises.

<u>Exemple 3 : Composition cosmétique de type émulsion huile-dans-eau colorée à base de colorants naturels</u>

| | | | |
|---|---|---|---|
| A | - | Mélange tartrate de dialkyle (C14-15 linéaire), alcool cétylstéarylique, alcool laurylique oxyéthylène (25 OE) oxypropylène (2) (COSMACOL PSE de SASOL) | 1,500 |
| | - | Mélange mono/distéarate de glycéryle/stéarate de polyéthylène glycol (100 OE) (ARLACEL 165 FL d'UNIQUEMA) | 2,000 |
| | - | Alcool stéarylique (C18 95 %) (LANETTE 18 de COGNIS) | 1,000 |
| | - | Cyclohexa diméthylsiloxane (DOW CORNING 246 FLUID de DOW CORNING) | 7,500 |
| | - | Isoparaffine (6-8 moles d'isobuthylène) hydrogénée (PARLEAM de NOF CORPORATION) | 2,500 |
| | - | P-hydroxybenzoate de butyle (distribué par UENO FINE | 0,150 |
| | - | CHEMICALS) 4-méthoxycinnamate de 2-éthyl hexyle protégé (PARSOL MCX de DSM NUTRITIONAL PRODUCTS) | 1,000 |
| B1 | - | Eau | 50,000 |
| | - | P-hydroxybenzoate de méthyle (distribué par NIPA) | 0,250 |
| | - | Chlorphénésine (de DENK (MERCK)) | 0,250 |
| B2 | - | Eau | 3,615 |
| | - | Extrait de betterave (Beetroot juice P-WS (4/96) de LCW (SENSIENT) | 13,760 |
| | - | Extrait de béta-carotène (B-Carotene 10 % P-WS) de LCW (SENSIENT) | 2,190 |
| | - | Sel de sodium de chlorophyline cuivrique en poudre (97/3) de LCW (SENSIENT)) | 0,035 |
| C | - | Eau | 1,000 |
| | - | Digluconate de chlorhexidine en solution (chlorhexidine digluconate 20% solution BP/PH. EUR. de SCHUTZ DISHMAN BIOTECH) | 0,250 |
| D | - | Eau | 9,750 |
| | - | Hyaluronate de sodium (PM : 1.100.000) en poudre (CRISTALHYAL de SOLIANCE) | 0,250 |
| E | - | Amidon de maïs estérifié par anhydride octénylsuccinique, sel d'aluminium (DRY FLO PLUS (28-1160) (EUROPE) de NATIONAL STARCH) | 3,000 |

Le mode opératoire est le même que celui de l'exemple 1.

Cette base de coloration permet d'obtenir sur une peau foncée une teinte bronzée.

Exemple 4 : Composition de type fond de teint sous forme d'émulsion solide

| | | | |
|---|---|---|---|
| A1 | - | Cire de candelilla purifiée (NC 1630 de CERA RICA NODA) | 1,800 |
| | - | Cire minérale d'hydrocarbures (C20/C60) (Ozokérite wax SP 1020 P de STRAL & PITSCH) | 1,200 |
| | - | P-hydroxybenzoate de propyle (de NIPA (CLARIANT)) | 0,300 |

(suite)

| | | | |
|---|---|---|---|
| | - | Poly méthylcétyl diméthyl méthylsiloxane oxyéthylène (20/75/5 - viscosité : 3000 CST) (Abil EM 90 de GOLDSCHMIDT (DEGUSSA)) | 2,000 |
| | - | Isononanoate d'isotridecyle (Crodamol TN (J) (ES02976) de CRODA)) | 9,000 |
| | - | Iso-stéarate polyglycérole (4 moles) (Isolan GI 34 de GOLDSCHMIDT DEGUSSA) | 1,500 |
| A2 | - | Ethers de dodécanédiol (2,2 moles) et de polyéthylène glycol (45 OE) (Elfacos ST 9 de AKZO NOBEL) | 4,000 |
| A3 | - | Cyclopentadiméthylsiloxane (DOW CORNING 245 fluid de DOW CORNING) | 28,625 |
| | - | Mélange de polydiméthylsiloxane oxyéthyl oxypropylène (18 OE/18 OP), de Cyclopentadiméthylsiloxane et d'eau (10/88/2) (DOW CORNING 5225C formulation AID de DOW CORNING) | 2,000 |
| A4 | - | Micro-billes de silice (SB150 de MIYOSHI KASEI) | 9,000 |
| | - | Silice pyrogénée à caractère hydrophobe (Aerosil R 972 de LCW (SENSIENT)) | 0,500 |
| | - | Microsphères creuses de poly méthacrylate de méthyle (granulométrie : 10 à 12 microns) (Covabead LH 85 de LCW (SENSIENT)) | 3,000 |
| B1 | - | Eau | 23,125 |
| | - | P-hydroxybenzoate de méthyle (de NIPA) | 0,400 |
| | - | Chlorphénésine (de DENK (MERCK)) | 0,300 |
| | - | Phénoxy-2 éthanol (de SEPPIC) | 0,500 |
| | - | Sulfate de magnésium, 7 H2O (de MERCK) | 1,000 |
| | - | Glycérol (Croderol GV 9000) (de CRODA) | 5,000 |
| | - | 1,3-butylène glycol | 3,000 |
| B2 | - | Eau | 3,000 |
| | - | Sel di-sodique de tartrazine (CI : 19140) (08005 FD et C Yellow 5 de LCW (SENSIENT)) | 0,255 |
| | - | Sel di-sodique du jaune brillant FCF (CI : 15985) (37001 FD & Yellow N°6 de UNIVAR) | 0,302 |
| | - | Sel di-sodique de vert brillant FCF (CI : 42053) (06503 FD & Green 3 de LCW (SENSIENT)) | 0,035 |
| | - | Sel di-sodique de fuchsine acide D (CI: 17200) (K7057 D et C Red 33) (LCW (SENSIENT) | 0,158 |

Mode opératoire :

La phase A1 est pesée dans un poêlon et fondue jusqu'à limpidité du mélange. Celui-ci est agité à la turbine et la température du bain d'huile est baissée jusqu'à environ 80 °C.

La phase A2 est ajoutée.

Les pigments de la phase A3 sont mouillés avec les huiles, puis broyés à la tricylindre en 3 passages. Le broyat est incorporé sous agitation dans le mélange (A1 + A2) légèrement refroidi (température inférieure à 80 °C). La pâte pigmentaire est laissée sous agitation pendant environ 30 minutes.

Lorsque le mélange (A1 + A2 + A3) est stabilisé à une température de 68 °C, les phases A4, puis A5 sont introduites. L'homogénéisation mécanique est maintenue pendant 10 minutes.

Parallèlement, la phase aqueuse B est préparée dans un bécher de 400 millilitres, à une température de 68 °C.

L'émulsion est réalisée à 67-68 °C sous agitation en versant lentement la phase aqueuse dans la phase grasse. L'agitation est maintenue pendant 10 minutes.

L'émulsion est ensuite coulée à 68 °C puis mise à l'étuve à 20 °C pendant 24 heures.

Ce produit permet d'obtenir sur une peau foncée une teinte marron neutre.

## Revendications

1. Procédé de maquillage d'une peau foncée comprenant l'application sur ladite peau d'une composition comprenant dans un milieu physiologiquement acceptable au moins un système de coloration directe à l'état soluble, ladite composition possédant en outre une viscosité supérieure à 100 centipoises et un pouvoir couvrant, défini par le Contrast Ratio, inférieur à 80.

2. Procédé de maquillage d'une peau foncée comprenant l'application sur ladite peau d'une composition comprenant dans un milieu physiologiquement acceptable au moins un système de coloration directe à l'état soluble, ladite composition possédant un pouvoir couvrant, défini par le Contrast Ratio, inférieur à 80 et un pH supérieur à 5.

**3.** Procédé selon l'une quelconques des revendications précédentes, dans lequel la composition possède une viscosité supérieure à 100 centipoises, notamment supérieure à 300 centipoises.

**4.** Procédé selon l'une quelconques des revendications précédentes, dans lequel ladite composition possède un pouvoir couvrant, défini par le Contrast Ratio, inférieur à 80, en particulier inférieur ou égal à 50.

**5.** Procédé selon l'une quelconque des revendications précédentes dans lequel le système de coloration directe est formé d'agent(s) de coloration hydrosoluble(s), liposoluble(s) ou d'un de leurs mélanges.

**6.** Procédé selon l'une quelconque des revendications 1 à 5, dans lequel ledit système de coloration directe comprend au moins un colorant choisi parmi le DC Red 17, le DC Red 21, le DC Red 27, le DC Green 6, le DC Yellow 11, le DC Violet 2, le DC Orange 5, le rouge Soudan, les carotènes, les xanthophylles, l'huile de palme, le brun Soudan, le jaune quinoléine, le rocou, le curcumin, le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétamine, le carmin, la chlorophylline cuivrique, le bleu de méthylène, les anthocyanines, la riboflavine, la juglone, la lawsone, le caramel, les extraits de soja fermenté, d'algues, de champignons, de micro-organismes, les sels de Flavylium non substitués en position 3, les extraits de Gesneria Fulgens, Blechum Procerum, du Saxifraga et les pigments susceptibles d'être obtenus par extraction par un solvant organique ou hydro-organique d'un milieu de culture de micromycètes du type monascus Monascus.

**7.** Procédé selon l'une quelconque des revendications précédentes, dans lequel ledit système de coloration directe comprend au moins un agent de coloration naturel.

**8.** Procédé selon l'une quelconque des revendications précédentes, dans lequel la composition comprend de 0,001 à 5 %, notamment de 0,01 à 3 % et en particulier de 0,025 à 1 % en poids d'agent(s) de coloration formant le système de coloration directe.

**9.** Procédé selon l'une quelconque des revendications précédentes, **caractérisé en ce que** ladite composition procure une variation de clarté $\Delta L$ négative variant de -20 à -1 et une variation de saturation $\Delta C^*$ variant de 0,5 à 20.

**10.** Procédé selon l'une quelconque des revendications précédentes, dans lequel ladite composition comprend au moins une phase grasse.

**11.** Procédé selon l'une quelconque des revendications précédentes, dans lequel ladite composition comprend au moins une phase aqueuse.

**12.** Procédé selon l'une quelconque des revendications 1 à 10, dans lequel ladite composition est anhydre.

**13.** Procédé selon la revendication 10, 11 ou 12, dans lequel ladite phase grasse contient au moins un corps gras liquide à température ambiante et à pression atmosphérique et/ou au moins un corps gras solide à température ambiante et à pression atmosphérique.

**14.** Procédé selon la revendication 13, dans lequel ledit corps gras liquide à température ambiante et à pression atmosphérique comprend au moins une huile volatile ou non volatile ou un de leurs mélanges.

**15.** Procédé selon la revendication 13 ou 14, dans lequel ledit corps gras solide à température ambiante et pression atmosphérique est choisi parmi les cires, les corps gras pâteux, les gommes et leurs mélanges.

**16.** Procédé selon l'une quelconque des revendications précédentes, dans lequel ladite composition comprend moins de 15 % en poids de charge(s).

**17.** Procédé selon l'une quelconque des revendications précédentes, dans lequel ladite composition comprend en outre au moins des pigments et/ou des nacres à l'état dispersé.

**18.** Procédé selon l'une quelconque des revendications précédentes, dans lequel ladite composition se présente sous une forme fluide de type liquide, pâte, émulsion directe ou inverse, ou gel.

**19.** Procédé selon l'une quelconque des revendications 1 à 17, dans lequel ladite composition se présente sous une

forme solide notamment compacte, pulvérulente ou coulée ou sous forme de stick.

**20.** Procédé selon l'une quelconque des revendications précédentes, dans lequel ladite composition se présente sous la forme d'un fond de teint à appliquer sur le visage ou le cou, d'un produit anti-cernes, d'un correcteur de teint, d'une crème teintée ou d'une base de maquillage pour le visage ou d'une composition de maquillage pour le corps.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9415580 A
- WO 9636309 A
- WO 9633690 A
- WO 9636323 A
- FR 2754708 A
- EP 1433460 A
- EP 1433462 A
- EP 1172091 A
- EP 1086683 A
- EP 749746 A
- EP 923928 A
- EP 930060 A


### Littérature non-brevet citée dans la description


- **KIRK-OTHMER.** Encyclopedia of Chemical Technology. WILEY, 1979, vol. 22, 333-432